# EUROPEAN PATENT SPECIFICATION

(11) **EP 0 483 216 B1**
(45) Date of publication and mention of the grant of the patent: **25.01.1995**
(21) Application number: 90911028.0
(22) Date of filing: 17.07.1990
(51) Int. Cl.: C12N 5/08, A61K 39/395, A61K 35/14

(54) **PREPARATION OF HUMAN ADHERENT LYMPHOKINE-ACTIVATED KILLER (A-LAK) CELLS**
VERFAHREN ZUR HERSTELLUNG MENSCHLICHER ADHÄRENTER LYMPHOKIN-AKTIVIERTER KILLERZELLEN (ALAK)
PREPARATION DE CELLULES K (A-LAK) ACTIVEES PAR LA LYMPHOKINE, ADHERENTES ET HUMAINES

(30) Priority: 21.07.1989 US 384134
(43) Date of publication of application: 06.05.1992
(73) Proprietor: TERUMO KABUSHIKI KAISHA, Tokyo 151 (JP)
(72) Inventor: LEUNG, Kam, Hung, Brookhaven, PA 19015 (US)
(74) Representative: von Kreisler, Alek, Dipl.-Chem.
(86) International application number: US9003900
(87) International publication number: WO9101369

(56) References cited:
- EP-A- 0 247 613
- EP-A- 0 257 962
- WO-A-88/01642
- Cancer Research, vol. 46, June 1986, Maureen Hoyer et al.:"Charactrization and modulation of human lymphokine (Interleukin 2) Activated Killer Cell Induction", pages 2834-2838
- Cancer Immunol Immunother, vol. 29, 1989, Robert J. Melder et al.:"Large-scale preparation of adherent lymphokine-activated killer (A-Lak) cells for adoptive immunotherapy in man", page 67-73
- The journal of immunology, vol. 142, no. 5, March 1989, Yoshio Gunji et al.:"Generation and characterization of purified adherent lymphokine activated killer cells in mice". page 1748-1754
- Cancer Research Vol., 48, June 1988, Robert J. Melder t al.:"A new Approach to generatin antitumor effectors for adoptive immunotherapy using human adherent lymphokine-activated killer cells", page 3461-3469
- The journal of immunology, vol. 134, no. 2, february 1985, Dwain L. Thiele et al.:"Modulation of human natural killer cell function by L.leucine methyl ester: monocyte-dependent depletion from human peripheral blood monounclear cells, page 786-793

## Description

### Background of the Invention

Natural killer (NK) cells and lymphokine-activated killer (LAK) cells have been implicated in immuno-surveillance against tumor cells (Barlozzari et al. (1983) J. Immunol. 131:1024; Rayner et al. (1985) Cancer 55:1327). It has been reported that the systemic administration of autologous LAK cells and IL-2 to patients with certain advanced cancers is beneficial (Rosenberg (1988) Immunology Today 9:58). LAK cells were prepared at concentrations of up to, but not exceeding, 1x10⁶ cells/mL. The process of preparing LAK cells for cancer therapy is very cumbersome and tedious since a large number of cells are needed per treatment for each patient. Also, the treatment of cancer patients by LAK/IL2 therapy is accompanied by significant toxicities (Rosenberg et al. (1987) N. Engl. J. Med. 316:889).

It has been previously shown that monocytes interfere with the generation of LAK activity by IL-2 (Hoyer et al. (1986) Cancer Res. 46:2834). L-leucine methyl ester (LeuOMe) and L-phenylalanine methyl ester (PME) were shown to remove monocytes from human peripheral blood mononuclear cells (PBMC) (Thiele and Lipsky (1985) J. Immunol. 134:786; Hoyer et al. (1986) Cancer Res. 46:2834). Furthermore, LeuOMe also depleted NK activity and NK cells (Thiele and Lipsky (1985) J. Immunol. 134:786; Hoyer et al. (1986) Cancer Res. 46:2834). We have shown that depletion of monocytes by PME allows generation of LAK cells by rIL-2 at cell densities of 5x10⁶ cells/mL or higher EP-A-0 247 613, published December 2,1987, and allowed U.S. application 07/038361, filed April 20, 1987).

It has been reported recently that a highly enriched LAK cell population can be obtained by selecting "adherent" LAK (A-LAK) cells, which can be isolated by adherence to plastic of 24-hr IL-2-activated and monocyte-depleted PBMC (Melder et al. (1988) Cancer Res. 48:346). These cells are highly proliferative and cytotoxic, and are enriched for Leu 19-positive cells; Leu 19 expression correlates with NK and LAK activity. The potential advantages and importance of A-LAK cells for adoptive immunotherapy in humans are: A-LAK cell preparations are relatively enriched for LAK cells with higher levels of antitumor activity; A-LAK cells may be effective in doses containing lower numbers of cells than conventional LAK therapy; less IL-2 may be needed for co-administration with A-LAK cells to patients, thereby reducing the toxicity of the therapy.

In previous procedures to prepare A-LAK cells, monocytes are removed by their adherence to nylon-wool columns (Melder et al., Cancer Immunol. Immunother. (1989) 29:67-73) or by centrifugal elutriation in order to generate A-LAK cells (Melder et al. (1988) Cancer Res. 48:34). These procedures for monocyte removal are tedious and complicated. Also, some LAK cell precursors may adhere to the nylon-wool columns, reducing the efficiency of the process to generate A-LAK.

### Summary of the Invention

The present invention provides an improved process for preparing adherent lymphokine activated killer (A-LAK) cells. The process comprises:
a. treating peripheral blood mononuclear cells (PBMC) to deplete monocytes;
b. culturing the remaining cells in culture medium containing IL-2 to generate lymphokine-activated killer (LAK) cells, in a plastic container to which a portion of the LAK cells adhere;
c. removing the non-adherent cells;
d. culturing the adherent LAK cells in medium conaining IL-2 to expand the adherent LAK cells.
The improvement comprises in step a depleting monocytes to 1-19% of total cells by treating the PBMC with an L-phenylalanine methyl ester (PME) or phenylalaninamide.

### Description of the Preferred Embodiments

The use of PME and other lower alkyl amino acid esters in a process to prepare LAK cells is disclosed in EP-A-0 247 613, published December 2, 1987, and allowed U.S. application 07/038361, filed April 20, 1987.

We have employed PME at concentrations of 1 to 5 mM as a single step for monocyte depletion. We have discovered that it is possible to generate A-LAK from PME-treated PBMC.

We have found that treatment of PBMC with PME prior to culturing the A-LAK cells in plastic containers, results in a substantial increase in the level of expansion of the A-LAK cells, relative to the expansion in cell number obtained in the absence of PME treatment. During the expansion of the A-LAK cells, following treatment with PME, the LAK cell functional cytolytic activity remains high. It was also discovered that the optimal concentration of PME used in the process, in terms of maximizing A-LAK cell expansion, depended on the cell density at which the A-LAK cells were first cultured in plastic containers. At a cell concentration of 2x10⁶ cells/mℓ,the optimal PME concentration was 1 mM; at a cell density of 1x10⁷ cells/mℓ,the optimal PME concentration was 5 mM.

The results suggest that PME depletes a subset of PBMC that inhibit the expansion of A-LAK cells and that, at higher PME concentrations, a separate PBMC subset, which enhances A-LAK cell expansion, is depleted.

The present invention involves an improved process for the expansion and enrichment of Leu 19-positive lymphocytes with high lytic activity. PBMC are first treated with PME to remove monocytes and other PME-sensitive cells. The resulting lymphocytes are incubated with rIL-2 in plastic flasks for 1 to 2 days. The adherent cells are then cultured for 8 to 21 days to obtain an increase in cell number and LAK activity. The cell densities used during the A-LAK preparation process, including the monocyte depletion step, are preferably 5x10⁶ to 2x10⁷ cells/mℓ,more preferably 1x10⁷ to 2x10⁷ cells/mℓ.

We have also discovered that A-LAK cells can be generated from PBMC treated with phenylalaninamide (PheNH₂). Our procedure using PME provides several substantial differences and important advantages over the procedures previously used to prepare A-LAK cells (Melder et al. (1988) Cancer Research 48:3461-3469). First, we used PME as a chemical agent to deplete monocytes from PBMC. This procedure can be better controlled by PME concentration and time of incubation with PME than can be achieved using the nylon-wool procedure. PME treatment of PBMC is also much more convenient and less time consuming to use than centrifugal elutriation which requires an elaborate setup, sterilization procedure, and the running of the elutriation. Second, in the process of the invention, the lymphocytes are cultured in plastic containers at a cell density of 1 to 2x10⁷/mℓ, as compared with 2x10⁶/mℓ which has been used previously. Thus, the present process will reduce the total volume of medium used by 5 to 10-fold, relative to previous procedures for the preparation of A-LAK cells. Third, the high cell density (1x10⁷/mℓ) generally gave more reliable expansion than low cell density (1x10⁶/mℓ). Fourth, enriched A-LAK cells can be generated from PBMC completely depleted of monocytes, but they are not as proliferative. The amount of monocyte depletion can be precisely controlled by the PME concentration used to treat the PBMC. As described above, the level of monocyte depletion cannot be readily controlled using elutriation and/or the nylon-wool column method of depleting monocytes.

A-LAK cells can be detached from plastic flasks and transferred to DuPont SteriCell® bags (DuPont) for culture. A-LAK cells can be generated and cultured in serum-free medium, such as AIM-V. The procedure employing PME-treated PBMC lends itself well to a process where relatively large volumes of blood and cell numbers are required, as is the case for adoptive immunotherapy. Furthermore, the A-LAK cells are highly enriched for the cytotoxic and proliferative cells. The use of A-LAK cells in adoptive immunotherapy should give a better understanding of the importance of LAK cells in the immunosurveillance of tumor cells and therapy for human cancer.

### Example 1

We have studied the generation of A-LAK cells from PBMC treated with PME. PBMC were treated with 1 or 5 mM PME and cultured at 2x10⁶ cells/mℓ or 1x10⁷ cells/mℓ with rIL-2 for 1 day in plastic flasks (Table 1). Nonadherent (NA) cells were removed and cells adherent to flasks were cultured with the conditioned media containing rIL-2 for 8 days. The resultant A-LAK cells were cytotoxic against ⁵¹Cr-labeled K562 and Raji target cells. A-LAK cells are also highly proliferative and have a high percent composition of the Leu-19 phenotype of NK and LAK cells. At the low cell density (2x10⁶ cells/mℓ), partial depletion of monocytes by 1 mM PME resulted in greater levels of cellular expansion of A-LAK cells than were obtained using 5mM PME. At the high cell density (1x10⁷ cells/mℓ), 5mM PME resulted in higher levels of cellular expansion than did 1 mM PME. Higher cellular expansion levels were observed with the 1 mM PME-treated PBMC, at both high and low cell densities, than with the untreated PBMC. Our results demonstrate that PME treatment of PBMC provides an efficient means to generate A-LAK cells for adoptive immunotherapy.

**Table 1**

| Generation of A-LAK Cells from PBMC Treated with Various PME Concentrations | | | | | |
|---|---|---|---|---|---|
| Cell Density | [PME] (mM) | % Monocytes Before Culture | LAK Activity (% Lysis) | % Leu 19+ | Fold Expansion |
| 2x10⁶/mℓ | 0 | 23 | 62.6 | 43 | 9 |
| 2x10⁶/mℓ | 1 | 19 | 63.4 | 44 | 85 |
| 2x10⁶/mℓ | 5 | 2 | 56.7 | 41 | 16 |
| 1x10⁷/mℓ | 0 | 23 | 20.8 | 15 | 5 |
| 1x10⁷/mℓ | 1 | 19 | 61.8 | 49 | 23 |
| 1x10⁷/mℓ | 5 | 2 | 55.4 | 60 | 36 |
| PBMC were treated with 0, 1, or 5 mM PME for 40 min. at a concentration of 1x10⁷ cells/mℓ. The various cell preparations (30 mℓ) were then incubated with 400 U/mℓ of rIL-2 at total cell densities of 2x10⁶ cells/mL or 1x10⁷ cell/mℓ in T75 flasks overnight. The culture medium containing non-adherent cells was removed, the non-adherent cells were separated from the medium, the medium was returned to the flasks, and the adherent (A) cells were cultured. The data shown are for cells cultured for 8 days. E:T ratio shown is 5:1 for LAK activity against Raji. | | | | | |

### Example 2

PBMC were treated with 5 mM PME and incubated with 400 U/mℓ of rIL-2 overnight at a cell density 2x10⁶/mℓ, 5x10⁶/mℓ or 1x10⁷/mℓ. IL-2 units are in Biological Response Modifier Program (BRMP) units (U). Non-adherent cells were decanted from the flasks. The adherent cells in the flasks were washed and counted. Conditioned medium (100%) was added back to the flasks for culture. The number of cells that adhered to the flasks increased with the initial adherent cell density. After 10 days in culture, the LAK activity and % Leu 19-positive cells were essentially the same amongst the three adherent cell densities. However, the fold expansion was found to increase with increasing cell density used during the process.

**Table 2**

| Generation of A-LAK Cells from PME-Treated PBMC at Various Cell Densities | | | | |
|---|---|---|---|---|
| Cell Density | Total Adherent Cells | LAK Activity (% lysis) | % Leu 19+ | Fold Expansion |
| A-LAK | | | | |
| 2x10⁶/mℓ | 5x10⁵ | 74.6 | 81 | 31 |
| 5x10⁶/mℓ | 1.2x10⁶ | 77.2 | 78 | 80 |
| 1x10⁷/mℓ | 1.7x10⁶ | 74.4 | 77 | 158 |

| NA-LAK | | | | |
|---|---|---|---|---|
| 1x10⁶/mℓ | | 52.6 | 19 | 1.7 |
| PBMC were treated with 5 mM PME and incubated with 400 U/mℓ rIL-2 overnight at the various cell densities NA cells were cultured with 400 U/mℓ rIL-2 at 1x10⁶/mℓ. The culture medium containing non-adherent cells was removed, the non-adherent cells were separated from the medium, the medium was returned to the flasks, and the adherent (A) cells were cultured. The data shown are for cells cultured for 10 days. E:T ratio shown is 5:1 for LAK activity against Raji. | | | | |

### Example 3

We have found that L-phenylalaninamide (PheNH₂) also depletes monocytes without adversely affecting NK cells. The generation of A-LAK cells from PBMC treated with PheNH₂ was compared with that obtained using PME treatment. As summarized in Table 3, the % Leu 19-positive cells and LAK activity from PheNH₂-treated PBMC were only slightly less than those of PME-treated PBMC. However, the number of A-LAK cells at day 8 was generally much higher from the PheNH₂-treated PBMC than from the PME-treated PBMC. This result may be due to the fact that PheNH₂ at 5 mM is less effective than PME at 5 mM in depleting monocytes. Thus, a higher number of monocytes presumably remain in the PheNH₂-treated PBMC and, as we have shown in Example 1, this low level of monocytes (1 to 10% of the total cells) appears to be optimal for maximal A-LAK cellular expansion.

**Table 3**

| Effect of PheNH₂ on A-LAK Cell Generation | | | | | |
|---|---|---|---|---|---|
| Treatment | % Monocytes Before Culture | % Leu 19+ | Activity (% lysis) | Cells/mℓ At Day 8 | Expansion |
| PME | 7 | 83 | 79.4 | 4.7x10⁵ | 22.3 |
| PheNH₂ | 16 | 80 | 73.2 | 9.5x10⁵ | 13.0 |
| PME | 1 | 94 | 85.0 | 9.0x10⁵ | NT |
| PheNH₂ | 11 | 63 | 70.9 | 8.5x10⁵ | NT |
| PME | 2 | 76 | 80.5 | 3.7x10⁵ | 18.5 |
| PheNH₂ | 5 | 77 | 78.2 | 1.2x10⁶ | 35.6 |
| PME | 8 | 96 | 61.2 | 7.0x10⁵ | 13.3 |
| PheNH₂ | 12 | 85 | 51.9 | 1.4x10⁶ | 23.3 |
| PBMC were treated with 5 mM PME or 5 mM PheNH₂. The A-LAK were generated using a total cell density of the culture of 5x10⁶ cells/mℓ. E:T ratio shown is 5:1 for LAK activity against Raji. Data shown were from four different donors. NT = not treated. | | | | | |

Although the invention has been described with reference to the preferred embodiments, PME and PheNH₂, the procedures and conditions described are also applicable to use in the A-LAK process of the other esters and amides mentioned in the summary of the invention.

## Claims

1. In a method of activating and expanding cells for use in adoptive immunotherapy which comprises:
a. treating peripheral blood mononuclear cells (PBMC) to deplete monocytes;
b. culturing the remaining cells in culture medium containing IL-2 to generate lymphokine-activated killer (LAK) cells, in a plastic container to which a portion of the LAK cells adhere;
c. removing the non-adherent cells;
d. culturing the adherent LAK cells in medium containing IL-2 to expand the adherent LAK cells;
the improvement which comprises in step a depleting monocytes to 1-19% of total cells by treating the PBMC with an L-phenylalanine methyl ester or phenylalaninamide.

2. Process of claim 1 wherein in step a the PBMC at a cell concentration in the range of 1x10⁶ to 2x10⁷ cells per ml are treated with the amino acid ester or amide at a concentration in the range of 1 to 5 mM, and in step b the PBMC are cultured for 1 to 2 days at a cell concentration in the range of 1x10⁶ to 2x10⁷ cells per ml.

3. Process of claim 2 wherein in step b the PBMC are cultured in plastic flasks and in step d the adherent LAK cells are cultured in the same plastic flasks or in closed gas-permeable plastic bags in the medium from step b for 8 to 21 days.

4. Process of claim 3 wherein in step a the PBMC concentration is 2x10⁶ cells per ml and the cells are treated with phenylalanine methyl ester or phenylalaninamide at a concentration of 1 mM.

5. Process of claim 3 wherein in steps a and b the PBMC concentration is in the range of 5x10⁶ to 2x10⁷ cells per ml and in step a the cells are treated with phenylalanine methyl ester or phenylalaninamide at a concentration of 5 mM.

6. Process of claim 5 wherein in steps a and b the PBMC concentration is in the range of 1x10⁷ to 2x10⁷ cells per ml.

7. Process of claim 6 wherein in steps a and b the PBMC concentration is 1x10⁷ cells per ml.

## Patentansprüche

1. Verfahren zur Aktivierung und Vermehrung von Zellen zur Verwendung bei der passiven Immuntherapie, umfassend:
a. Behandeln peripherer einkerniger Blutzellen (PBMC), um Monocyten auszudünnen;
b. Kultivieren der übrigen Zellen in IL-2-haltigem Kulturmedium, um lymphokinaktivierte Killerzellen (LAK) zu erzeugen, in einem Kunststoffbehälter, an dem ein Teil der LAK-Zellen haftet;
c. Entfernen der nicht anhaftenden Zellen;
d. Kultivieren der anhaftenden LAK-Zellen in IL-2-haltigem Medium, um die anhaftenden LAK-Zellen zu vermehren;
umfassend in Schritt a das Ausdünnen der Monocyten auf 1-19% der gesamten Zellen durch Behandlung der PBMC mit einem L-Phenylalaninmethylester oder Phenylalaninamid.

2. Verfahren gemäß Anspruch 1, wobei in Schritt a die PBMC bei einer Zellkonzentration im Bereich von 1·10⁶ bis 2·10⁷ Zellen pro ml mit dem Aminosäureester oder -amid einer Konzentration im Bereich von 1 bis 5 mM behandelt werden und in Schritt b die PBMC 1 bis 2 Tage bei einer Zellkonzentration im Bereich von 1·10⁶ bis 2·10⁷ Zellen pro ml kultiviert werden.

3. Verfahren gemäß Anspruch 2, wobei in Schritt b die PBMC in Kunststoffflaschen kultiviert werden und in Schritt d die anhaftenden LAK-Zellen 8 bis 21 Tage in derselben Kunststoffflasche oder in geschlossenen gasdurchlässigen Kunststoffbeuteln im Medium von Schritt b kultiviert werden.

4. Verfahren gemäß Anspruch 3, wobei in Schritt a die PBMC-Konzentration 2·10⁶ Zellen pro ml beträgt und die Zellen mit Phenylalaninmethylester oder Phenylalaninamid einer Konzentration von 1 mM behandelt werden.

5. Verfahren gemäß Anspruch 3, wobei in Schritt a und b die PBMC-Konzentration im Bereich von 5·10⁶ bis 2·10⁷ Zellen pro ml liegt und in Schritt a die Zellen mit Phenylalaninmethylester oder Phenylalaninamid einer Konzentration von 5 mM behandelt werden.

6. Verfahren gemäß Anspruch 5, wobei in Schritt a und b die PBMC-Konzentration im Bereich von 1·10⁷ bis 2·10⁷ Zellen pro ml liegt.

7. Verfahren gemäß Anspruch 6, wobei in Schritt a und b die PBMC-Konzentration 1·10⁷ Zellen pro ml beträgt.

## Revendications

1. Un procédé d'activation et d'expansion des cellules pour une utilisation dans une immunothérapie adoptive qui comprend :
a. le traitement des cellules mononucléaires du sang périphérique (PBMC) pour entraîner la déplétion de monocytes;
b. la culture des cellules restantes dans le milieu de culture contenant de l'IL-2 pour générer des cellules tueuses activées par la lymphokine (LAK), dans un récipient en plastique auguel une partie des cellules LAK adhère;
c. élimination des cellules non adhérentes;
d. culture des cellules LAK adhérentes dans du milieu contenant IL-2 pour obtenir l'expansion des cellules LAK adhérentes;
caractérisé en ce qu'il comprend dans l'étape a. la déplétion des monocytes jusqu'à 1 à 19% des cellules totales par traitement des PBMC avec un méthyl ester de L-phénylalanine ou du phénylalaninamide.

2. Procédé selon la revendication 1, caractérisé en ce que dans l'étape a. les PBMC à une concentration cellulaire comprise dans un intervalle de 1 x 10⁶ à 2 x 10⁷ cellules/ml sont traités par l'amide ou l'ester d'acide aminé à une concentration comprise dans un intervalle de 1 à 5 mM, et dans l'étape b. les PBMC sont cultivés pendant 1 à 2 jours à une concentration cellulaire comprise dans un intervalle de 1 x 10⁶ à 2 x 10⁷ cellules/ml.

3. Procédé selon la revendication 2, caractérisé en ce que dans l'étape b. les PBMC sont cultivés dans des bouteilles en plastique et dans l'étape d. les cellules LAK adhérentes sont cultivées dans les mêmes bouteilles en plastique ou dans des sacs en plastique clos perméables aux gaz dans le milieu provenant de l'étape b. pendant 8 à 21 jours.

4. Procédé selon la revendication 3, dans lequel dans l'étape a. la concentration en PBMC est 2 x 10⁶ cellules/ml et les cellules sont traitées par du méthyl ester de phénylalanine ou du phénylalaninamide à une concentration de 1 mM.

5. Procédé selon la revendication 3 dans lequel dans l'étape a. et dans l'étape b. la concentration en PBMC est comprise dans un intervalle de 5 x 10⁶ à 2 x 10⁷ cellules/ml et dans l'ètape a. les cellules sont traitées avec du méthyl ester de phénylalanlne ou du phénylalaninamide à une concentration de 5 mM.

6. Procédé selon la revendication 5, caractérisé en ce que dans les étapes a. et b. la concentration en PBMC est comprise dans un intervalle de 1 x 10⁷ à 2 x 10⁷ cellules/ml.

7. Procédé selon la revendication 6 dans lequel dans les étapes a. et b. la concentration en PBMC est de 1 x 10⁷ cellules/ml.
